# EUROPEAN PATENT APPLICATION

(11) **EP 3 441 472 A1**
(43) Date of publication of application: **13.02.2019**
(21) Application number: 18020041.2
(22) Date of filing: 30.01.2018
(51) Int. Cl.: C12P 5/02, A01G 33/00, C12M 1/00, C12M 1/107

(54) **MICRO ALGAE TO BIO METHANE**

(30) Priority: 07.08.2017 EP 17020340
(71) Applicant: Sukhmeet Singh Anand, 08870 Barcelona (ES)
(72) Inventor: Sukhmeet Singh Anand, 08870 Barcelona (ES)

(57) **Abstract**

**TECHNICAL FIELD**

The invention relates to efficient use and control of thermal energy and carbon dioxide in the process of producing microalgae for methane (CH4) production through fermentation.

The microalgae is grown in a controlled environment with the aid of light, carbon dioxide, CO2 and heat.

The invention includes a feature to store heat collected by the microalgae ponds during daytime.

The heat is used to keep the algae pond and fermentation tank at an optimal temperature for micro algae yield (25 to 30C) and the fermentation tank (32-40C).

An additional feature of the invention is that biogas is scrubbed using microalgae water solution for effective upgrading of the biogas to biomethane and carbon dioxide source for the microalgae growth.

## Description

MICROALGAE IS GROWN IN ENCLOSED PONDS (1) OR LARGE DIAMETER PLASTIC TUBES LAID OUT ON THE GROUND SUBJECTED TO SUNLIGHT. THE MICROALGAE GROWING REQUIRES SUNLIGHT, CARBON DIOXIDE (CO2), NUTRIENTS AND SOME TRACE ELEMENTS. NUTRIENTS ARE DOSED (11) TO THE POND CIRCULATING SYSTEM, INTO THE MICROALGAE WATER SOLUTION. THE WATER CONTAINING MICROALGAE IN THE PONDS, IS CONTINUOUSLY CIRCULATED TO AVOID SEDIMENTATION IN THE PONDS WHILE MAINTAINING A HOMOGENEOUS MIXTURE OF ADEQUATE LEVEL OF NUTRIENTS AND CO2 FOR GROWING MICROALGAE.

THE MICROALGAE PONDS ARE COVERED WITH THIN LIGHT PENETRATING MATERIAL (E.G. PLASTIC FILM) TO PREVENT WATER EVAPORATION FROM THE PONDS AND BIOLOGICAL CONTAMINATION OF THE ALGAE GROWTH MEDIA.

THERE ARE SEVERAL PARALLEL CONNECTED PONDS (1), TO ALLOW TAKING A POND OUT OF OPERATION FOR CLEANING AND SERVICE.

THE ALGAE POND (1) CAN BE EQUIPPED WITH ARTIFICIAL LIGHT FOR ALGAE PRODUCTION DURING LOW SUN LIGHT PERIODS.

THE MICROALGAE CULTURE IS A MULTI-SPECIE TESTED AND OPTIMIZED FOR LOCAL CLIMATIC CONDITIONS. A BROAD SELECTION OF SPECIES TO TARGET A ROBUST MICROALGAE CULTURE.

THE OPTIMAL TEMPERATURE SPAN FOR MICROALGAE GROWTH IS IN THE RANGE OF 25 - 35C, DEPENDENT UPON LOCAL SPECIES. AS THE PONDS ARE COVERED, TEMPERATURE CANNOT BE REGULATED BY EVAPORATION OF WATER BUT NEEDS TO BE DONE BY OTHER MEANS.
WATER FROM THE ALGAE SOLUTION TANK IS KEPT COLD BY COOLING IT AT NIGHT OR HEATING IT AT DAY WITH HEAT FROM THE PONDS OR AN ALTERNATIVE HEAT SOURCE. THE ALGAE SOLUTION POND IS TEMPERATURE CONTROLLED BY MEANS OF HEATING OR COOLING. FURTHERMORE, THE FERMENTATION TANK (4) CAN BE HEATED OR COOLED BY THE SAME SYSTEM.
THE ALGAE SOLUTION RESERVOIR (2) SERVES THE PURPOSE OF THERMAL STORAGE, SO WARM WATER IS AVAILABLE IF POND TEMPERATURE FALLS BELOW THE OPTIMAL TEMPERATURE WINDOW AND COLD WATER IS AVAILABLE IF THE TEMPERATURE OUT OF THE ALGAE POND (1) EXCEEDS THE OPTIMAL TEMPERATURE WINDOW.

A COOLER (13) CAN DISCHARGE EXCESS HEAT AND A HEATER CAN ADD HEAT IF REQUIRED, SUCH AS DURING NIGHT PERIOD.

IN A SECTION OF THE ALGAE SOLUTION RESERVOIR (2) THE HARVESTED MICROALGAE IS PUMPED IN FROM THE ALGAE POND (1). THERE WILL BE SEDIMENTATION OF MICROALGAE IN THE BOTTOM OF THE ALGAE SOLUTION RESERVOIR (2). THE MICROALGAE CONCENTRATION OF THE HARVESTED MICROALGAE FROM THE ALGAE POND (1) IS IN THE RANGE OF 10 GR PER KG MICROALGAE WATER SOLUTION. FOR THE FERMENTATION PROCESS, A CONCENTRATION OF 100 GR PER KG IS REQUIRED. THEREFORE, THE MICROALGAE SOLUTION MUST BE CONDITIONED BEFORE ENTERING THE FERMENTATION TANK. THE CONDITIONING TAKES PLACE IN THE ALGAE CONDITIONER (3).

ARTIFICIAL LIGHT CAN OPTIONALLY BE INSTALLED IN THE ALGAE SOLUTION RESERVOIR (2) FOR MICROALGAE GROWTH DURING LOW SUN LIGHT CONDITIONS.
THE MICROALGAE NEEDS CO2 TO GROW. PART OF THE CO2 IS PROVIDED FROM THE CO2 REMOVAL BY WAY OF SCRUBBING OF THE PRODUCED BIOGAS (4), THE OTHER PART FROM AERATING THE ALGAE SOLUTION RESERVOIR (2) BY AN AIR FAN (12).

POTENTIALLY, SUGAR OR STARCH MAY BE DOSED TO THE ALGAE SOLUTION TANK FOR MICRO ALGAE PRODUCTION BASED NON-PHOTOSYNTHESIS MICROALGAE SPECIES, WHICH CAN INCREASE MICROALGAE PRODUCTION DURING PERIODS WITHOUT SUNLIGHT.

THE ALGAE CONDITIONING PLANT (3), SHALL DE-WATER THE MICROALGAE WATER SOLUTION TO A DRY MATTER CONTENT OF APPROXIMATE 100 GR PER KG. FURTHERMORE, THE ALGAE CONDITION (3) MAY DISINTEGRATE THE MICROALGAE BY THERMAL OR MECHANICAL PROCESS TO MAKE FAT AND PROTEINS EASILY ACCESSIBLE FOR THE FERMENTATION BACTERIA.
THE DEWATERING MAY TAKE PLACE BY WATER CYCLONES, FILTER OR SIMILAR EQUIPMENT.

IN THE FERMENTATION TANK (4), THE BIOGAS PRODUCTION TAKES PLACE AT MESOPHILIC CONDITIONS AT TEMPERATURES IN THE RANGE OF 32-40C. THE FERMENTATION TANK (4) CAN BE COOLED WITH THE COOLING CIRCUIT OR HEATED WITH EXCESS HEAT FROM THE ALGAE PONDS (1) OR OTHER SOURCES OF HEAT (14). THE RESIDENCE TIME IN THE FERMENTATION TANK IS 7 - 10 DAYS, BASED ON OPTIMAL BIOGAS YIELD. FOR SOME MICROALGAE SPECIES, THE OPTIMAL RESIDENCE TIME MAY BE DIFFERENT. THERMOPHILIC CONDITIONS (50-55C) IN THE FERMENTATION TANK WILL ONLY BE APPLIED IF THE BIOGAS IS USED FOR ELECTRICAL ENERGY PRODUCTION AND THE WASTE HEAT IS AVAILABLE FOR HEATING. THERMOPHILIC CONDITIONS WILL REDUCE THE RESIDENCE TIME TO 2 - 5 DAYS, BUT WILL REQUIRE A TWO-STAGE FERMENTATION TANK WITH LOWER TEMPERATURE HYDROLYSIS PROCESS AND A HIGHER TEMPERATURE METHANISATION PROCESS.

THE PRODUCED BIOGAS SITS IN THE FERMENTATION TANK GAS DOME (5) BEFORE H2S IS REMOVED BY A FILTER BASED FE2O3 (6) OR ANOTHER ACTIVE AGENT.

THE CO2 IN THE BIOGAS IS REMOVED BY SCRUBBING THE GAS WITH MICROALGAE WATER SOLUTION FROM THE ALGAE SOLUTION RESERVOIR (2). THE UPGRADED BIOGAS IS COMPRESSED AND DRIED (8) BEFORE BEING EXPORTED.

IN CASE OF ELECTRICAL POWER GENERATION, H2S FILTERING (5), CO2 SCRUBBING (4) AND GAS COMPRESSION (9) ARE NOT NECESSARY AND CAN BE LEFT OUT. THE BIOGAS WILL HAVE TO BE CONDITIONED FOR ENGINE USED (DRIED).

IN CASE THE BIOGAS IS USED FOR ELECTRICAL ENERGY GENERATION AS FUEL FOR A GAS ENGINE, THE FLUE GAS FROM THE MOTOR WILL BE SCRUBBED WITH MICROALGAE SOLUTION WATER FROM THE ALGAE SOLUTION RESERVOIR (2) IN ORDER TO RECOVER THE CO2 IN THE FLUE GAS. HEAT RECOVERED FROM THE FLUE GAS OR ENGINE COOLING CAN BE USED FOR CONDITIONING THE TEMPERATURES IN THE ALGAE POND (1), ALGAE CONDITIONER (3) AND FERMENTATION TANK (6)

SLUDGE FROM THE FERMENTATION TANK (5) IS EXTRACTED IN THE BOTTOM OF THE TANK AND DEWATERED (9) BEFORE THE SLUDGE IS DISCHARGED FROM THE PLANT. THE WASTE WATER FROM THE DEWATERING PROCESS IS CLEANED AND REUSED AND PUMPED TO ALGAE SOLUTION RESERVOIR (2).

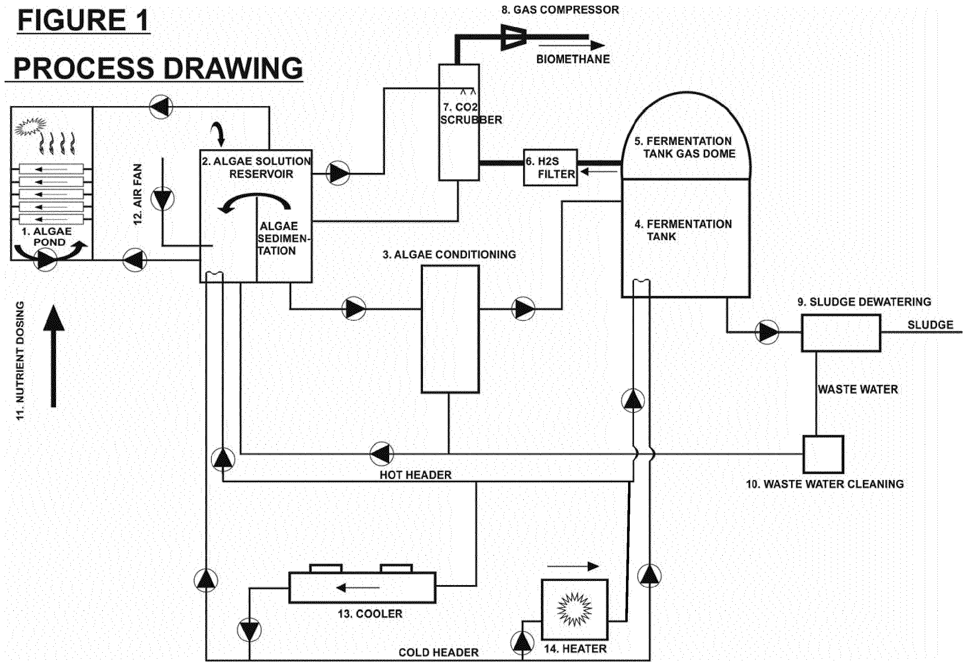

### EXPLANATION OF FIGURE 1

1) MICROALGAE PONDS CONNECTED IN PARALLEL WITH CIRCULATION PUMP AND PIPING. THE PONDS ARE COVERED WITH TRANSPARENT PLASTIC OR SIMILAR MATERIAL TO PREVENT WATER EVAPORATION AND ALLOW SUNLIGHT PENETRATION. HOT OR COLD WATER FROM THE MICROALGAE RESERVOIR CAN BE ADDED TO CONTROL THE TEMPERATURE OF THE MICROALGAE POND AND HARVEST THE MICROALGAE. EXCESS WATER IS LED BACK TO MICROALGAE RESERVOIR (2).
2) THE ALGAE RESERVOIR HOLDS THE HARVESTED MICROALGAE WHICH IS PUMPED FROM THE ALGAE POND (1) INTO THE ALGAE RESERVOIR. THE MICROALGAE SETTLES AND SEDIMENTS IN ONE AREA OF THE ALGAE RESERVOIR. CO2 IS ADDED TO THE WATER IN THE ALGAE RESERVOIR FROM THE BIOGAS SCRUBBING (7) OR FROM AERATING THE WATER MICROALGAE SOLUTION WITH THE AIR FAN (12). THE ALGAE RESERVOIR FUNCTIONS AS THERMAL STORAGE AND THE WATER MICROALGAE SOLUTION IS HELD AT A TEMPERATURE INTERVAL OF 20 - 30C. IF THE POND TEMPERATURE IS TOO LOW, THEN HIGH TEMPERATURE WATER MICROALGAE SOLUTION FROM THE RESERVOIR IS PUMPED TO THE ALGAE PONDS. IF THE TEMPERATURE IN THE ALGAE POND IS TOO HIGH, THEN COLD WATER FROM THE LOWER PART OF ALGAE RESERVOIR IS PUMPED TO THE ALGAE PONDS TO COOL IT DOWN.
3) THE SEDIMENTED MICROALGAE IN THE ALGAE RESERVOIR IS PUMPED TO THE ALGAE CONDITIONING SYSTEM, WHICH SHALL INCREASE DRY MATTER TO APPROXIMATE 100 GR PER LITER SOLUTION PUMPED TO THE FERMENTATION TANK (4). FURTHERMORE, DISINTEGRATION OF THE MICROALGAE BY THERMAL TREATMENT MAY TAKE PLACE TO MAKE FAT AND PROTEINS MORE AVAILABLE IN THE FERMENTATION TANK (4). THE DEWATERING OF WATER MICROALGAE SOLUTION MAY TAKE PLACE BY CYCLONES OR OTHER FILTER TECHNOLOGY.
4) FERMENTATION OF THE MICROALGAE WHICH IS GENERATING BIOGAS.
   THE SLUDGE / NON-CONVERTED MATERIAL IS COLLECTED IN THE BOTTOM OF THE FERMENTATION TANK AND PUMPED OUT. THE TEMPERATURE OF THE FERMENTATION TANK MUST BE CONTROLLED IN AN OPTIMAL TEMPERATURE RANGE OF 32-35C. HEAT COIL IN THE TANK MAKES IT POSSIBLE TO ADD HEAT OR EXTRACT HEAT IN ORDER MAINTAIN THE OPTIMAL TEMPERATURE IN THE FERMENTATION TANK.
   THE FERMENTATION TANK WILL HAVE A STIRRING MECHANISM TO MAINTAIN THE FLUID HOMOGENEOUS.
   TEMPERATURE SWINGS IN THE FERMENTATION TANK CAN LEAD TO SIGNIFICANT REDUCTION OF BIOGAS GENERATION RATE AND TAKE SEVERAL DAYS TO RESTORE.
5) THE GAS DOME ACCUMULATES THE GAS BUBBLES PRODUCED IN THE FERMENTATION TANK (4). THIS GAS DOME (5) IS MADE FROM A FLEXIBLE LINER AND IS INFLATED WITH THE PRODUCED BIOGAS AND ACTS AS A BUFFER FOR THE BIOGAS UPGRADING (6) AND (7).
6) H2S FILTER TO REMOVAL OF H2S BE FERRO OXIDES OR SIMILAR TECHNOLOGY.
7) THE CO2 SCRUBBER IS FOR REMOVING CO2 FROM THE BIOGAS IN ORDER TO INCREASE THE METHANE (CH4) CONTENT ABOVE 95% OR LEVEL REQUIRED TO EXPORT THE BIO METHANE. WATER MICRO ALGAE SOLUTION IS USED FOR SCRUBBING THE BIOGAS BRINGING THE CO2 DIRECTLY TO ALGAE POND SYSTEM WHERE IT IS CONSUMED TO PRODUCE MORE MICROALGAE.
8) GAS COMPRESSOR TO INCREASE PRESSURE OF THE BIOMETHANE TO EXPORT CONDITIONS
9) SLUDGE EXTRACTED FROM THE FERMENTATION TANK SHALL BE DEWATERED BEFORE BEING REMOVED FROM THE PLANT.
   DEWATERING IS BY FILTER PRESS OR SIMILAR TECHNOLOGY. WASTE WATER IS SENT TO WATER CLEANING (10)
10) WATER CLEANING BY SEDIMENTATION TANK AND SAND FILTER TO REMOVE PARTICLES. THE CLEANED WATER IS LED TO THE ALGAE RESERVOIR (2).
11) NUTRIENT DOSING TO MICROALGAE POND CIRCULATION SYSTEM TO SECURE CORRECT DOSING OF NITROGEN, POTASSIUM, SODIUM AND TRACE METALS AS REQUIRED
12) FAN FOR BLOWING AIR INTO THE ALGAE RESERVOIR TO ADD ADDITIONAL CO2 IF REQUIRED.
13) COOLER TO REMOVE EXCESS HEAT FROM THE THERMAL SYSTEM
14) HEATER IN THE FORM OF A BOILER OR SOLAR COLLECTORS TO INJECT HEAT INTO THE THERMAL SYSTEM TO HEAT THE ALGAE RESERVOIR AND / OR THE FERMENTATION TANK (4)

## Claims

1. THERMAL INTEGRATION OF THE HEAT RECOVERY FROM THE ALGAE PONDS SECURES STABLE AND HIGH YIELD OF MICROALGAE PRODUCTION

2. CO2 SCRUBBING OF THE BIOGAS WITH MICROALGAE SOLUTION WATER INCREASES CO2 CONCENTRATION IN THE ALGAE PONDS AND INCREASE GROWTH RATE.

3. UTILIZING LOCAL MICROALGAE SPECIES WITH FOREIGN FOR ROBUST CULTURE

4. MICRO ALGAE SEDIMENTATION IN THE ALGAE SOLUTION RESERVOIR INCREASE MICROALGAE CONCENTRATION BY GRAVITY WITHOUT THE USE OF ENERGY.

5. ADDING SUGAR OR STARCH TO THE ALGAE SOLUTION RESERVOIR ENABLING NON-PHOTOSYNTHESIS GROWTH OF MICRO ALGAE.

6. USING ARTIFICIAL LIGHT IN THE ALGAE SOLUTION RESERVOIR FOR MICRO ALGAE PRODUCTION.
